# EUROPEAN PATENT APPLICATION

(11) **EP 1 616 550 A1**
(43) Date of publication of application: **18.01.2006**
(21) Application number: 04728658.8
(22) Date of filing: 21.04.2004
(51) Int. Cl.: A61J 3/00, G06F 17/30

(54) **MEDICINE MANAGEMENT SYSTEM**

(30) Priority: 23.04.2003 JP 2003118147
(71) Applicant: Yuyama Mfg. Co., Ltd., Toyonaka-shi, Osaka 561-0841 (JP)
(72) Inventor: NAKATA, Kiyoyuki c/o Yuyama MFG. CO., LTD., Toyonaka-shi, Osaka 5610841 (JP); SAWAKI, Masanori c/o Yuyama MFG. CO., LTD., Toyonaka-shi, Osaka 5610841 (JP)
(74) Representative: Selting, Günther
(86) International application number: PCT/JP2004/005708
(87) International publication number: WO 2004/093776

(57) **Abstract**

A storage means (2) stores medicine codes associated with respective medicines and combination modification information corresponding to a combination of medicine information rearranged according to the medicine codes, and
a combination adequacy judging means (1) rearranges the medicine information according to the medicine codes stored in the storage means (2) when two or more medicines are included in information of one or more prescriptions for a patient, and judges combination adequacy with reference to the combination modification information called from the storage means (2) according to obtained order and combination of the medicine information. This makes possible to appropriately judge the adequacy of the combination even when three or more medicines are combined.

## Description

### FIELD OF THE INVENTION

The present invention relates to a medicine management system allowing combination adequacy to be judged when two or more medicines are combined.

### BACKGROUND OF THE INVENTION

Conventionally, there has been proposed a method for determining whether or not there is a possibility that combined medicines are out of effective ranges of acid and alkali so as to allow combination adequacy to be judged in advance when a plurality of medicines are administered to a patient (see, e.g., JP 2002-113072 A). In this method, all the combinations of two medicines out of the medicines specified in prescription information are subjected to data search for finding out whether or not the combinations are included in an incompatibility file.

### Disclosure of Invention

In this method, however, combination adequacy is judged based only on the combinations of two medicines. Consequently, if three or more medicines are to be combined and consideration is given to their combination orders, then the number of combinations becomes enormous, thereby making the method impractical.

Morcover, information on combination modification when three or more medicines are combined is scarcely found in documents and therefore verification and confirmation thereof should often be performed on the spot. Furthermore, even if the results of the verification and confirmation operation are recorded, the result data needs to be checked by a person when the data is shared and utilized in a hospital or a pharmacy, and there has been no such system that automates this checking operation.

Moreover, as for the combination modification, in the case of combining, for example, a medicine A (Elemcnmic), a medicine B (PN Twin) and a medicine C (Solu-Cortef), any combinations of these two medicines do not cause combination modification, but there is a possibility that a combination of these three medicines may cause combination modification (combination modification is not generated in the combination order of the medicines A, B and C but is generated in the combination order of the medicines A, C and B). In this case, it is impossible to confirm the combination modification with a conventional system that checks the combination modification of two medicines.

An object of the present invention is to provide a medicine management system allowing combination adequacy to be judged appropriately when three or more medicines are combined.

In order to accomplish the object of the present invention, there is provided a medicine management system, including: storage means for storing medicine information on medicines and combination modification information showing change when a plurality of medicines are combined; and

combination adequacy judging means for judging combination adequacy based on the combination modification information scored in the storage means when two or more medicines are included, in information of one or more prescriptions for a certain patient, wherein

the storage means stores medicine codes associated with respective medicines and combination modification information corresponding to a combination of medicine information rearranged according to the medicine codes, and

the combination adequacy judging means rearranges the medicine information according to the medicine codes stored in the storage means when two or more medicines are included in the prescription information, and judges combination adequacy with reference to the combination modification information called from the storage means in conformity to obtained order and combination of the medicine information.

This structure eliminates the necessity of storing combination modification by every combination order of medicines, thereby making it possible to suppress a storage capacity necessary for the storage means. Moreover, in the case of combining two or more medicines, medicines are rearranged according to the medicine codes, and the combination modification information should be searched only in accordance with a combination of medicines stored in the storage means in the rearranged order, which allows high-speed search processing. This management system is particularly effective when the number of medicines to be combined are increased to three or more.

Further, in order to accomplish the object of the present invention, there is provided a medicine management system, including:
storage means for storing medicine information on medicines and combination modification information showing change when a plurality of medicines are combined; and
combination adequacy judging means for judging combination adequacy based on the combination modification information stored in the storage means when two or more medicines are included in prescription information, wherein
the storage means stores medicine related information on each medicine, calculates combinations of two or more medicines by a hash function based on the medicine related information, and stores combination modification information for every obtained hash values, and

When the prescription information includes two or more medicines, the combination adequacy judging means calculates hash values based on the medicine related information stored in the storage means and calls corresponding combination modification information from the storage means based on the obtained hash values to judge combinalion adequacy.

With this structure, allotting a medicine code to every medicine makes it possible to identify a combination of medicines to be combined and to search combination modification information only by performing calculation by a hash function based on the medicine codes to obtain hush values.

A hush function should be the function which returns different values when medicine combinations are different and which returns identical hush values when combinations are identical even if their orders are different. It is also possible to obtain a desired hush value by selecting medicine codes to be associated per medicine.

The storage means stores a master file in which unrewritable combination modification information is stored and a case card file in which combination modification information can be newly stored, which is preferable because combination modification information can be added accordingly based on clinical examples of combination modification obtained at medical scenes.

The medicine management system includes display means for displaying the combination adequacy judged in the combination adequacy judging means, wherein
combination modification information on combinations of all medicines displayed in the display means can be displayed, which is preferable because the combination modification can be understood at one view.

The combination modification information on combinations of all medicines displayed in the display means can be changed and can be newly stored in the case card file, which is preferable because the combination modification information can be added easily and thoroughly.

After a medicine registration screen allowing a plurality of combined medicines to be registered with respect to each combination unit is displayed on the display means, all combinations among all combination units can be displayed by a list on a combined medicine confirmation screen, and new combination modification information can be inputted and stored in the case card file, which is preferable because new registration of the combination modification information can be conducted easily and thoroughly.

When a stored combination of medicine information has combination modification due to difference in combination order, the storage means stores the change, and when a pertinent combination is referred, the medicine information is rearranged in an appropriate combination order and displayed on the display means, which is preferable because the data is more accessible.

When the prescription information includes two or more medicines, the combination, adequacy judging means selects medicines to be simultaneously administered based on procedure codes stored in the storage means before rearranging medicine information according to the medicine codes, which is preferable because it becomes possible to reduce the number of search targets compared to the case of simply judging combination adequacy of the medicines prescribed simultaneously to one person, thereby allowing search processing to be performed at higher speed.

### BRIEF DESCRIPTION OF THE DRAWINGS

Fig. 1 is a block diagram showing a medicine management system according to the embodiment;
Fig. 2 is a block diagram showing the procedures of combination modification check;
Fig. 3A is a view showing a screen listing injection audits;
Fig. 3B is a view showing a prescription input screen;
Fig. 3C is a view showing the prescription input screen of Fig. 3B In the state of scroll down;
Fig. 3D is a view showing a screen listing combination modification results;
Fig. 4 is a view showing a combination modification information screen;

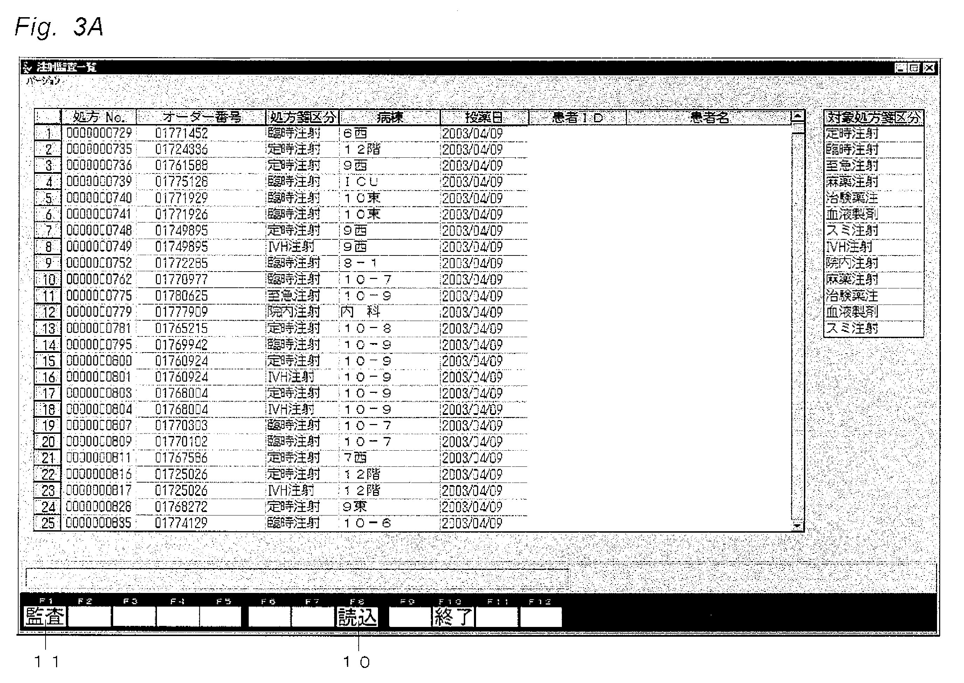

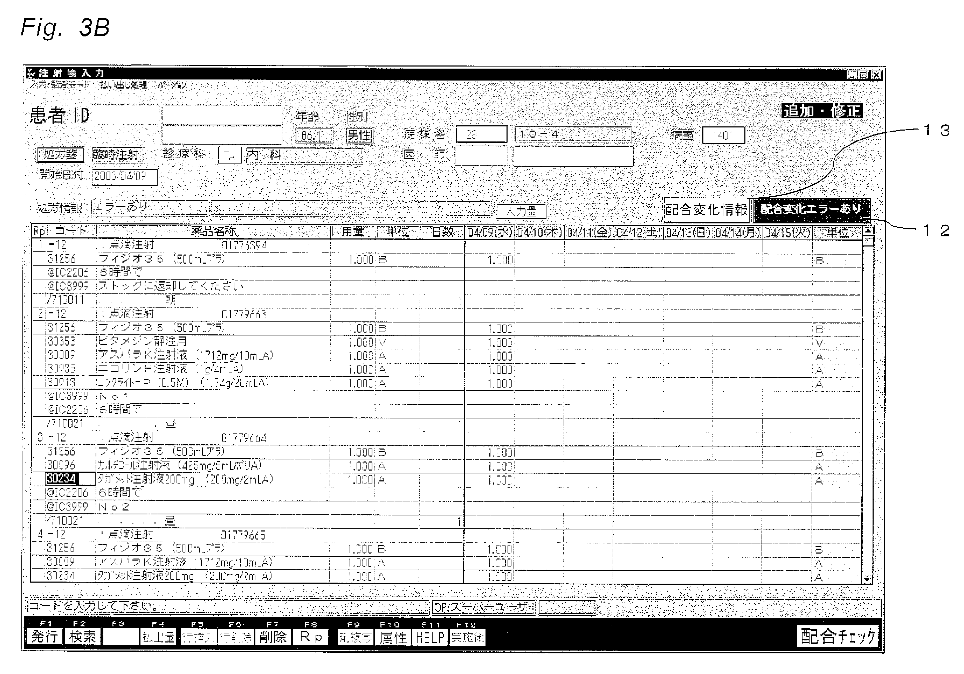

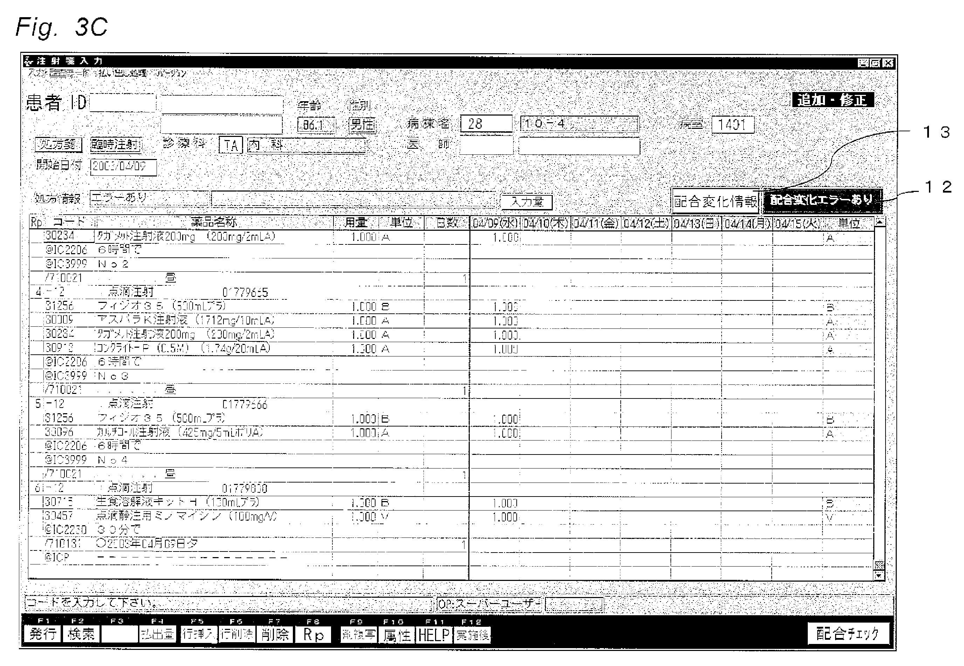

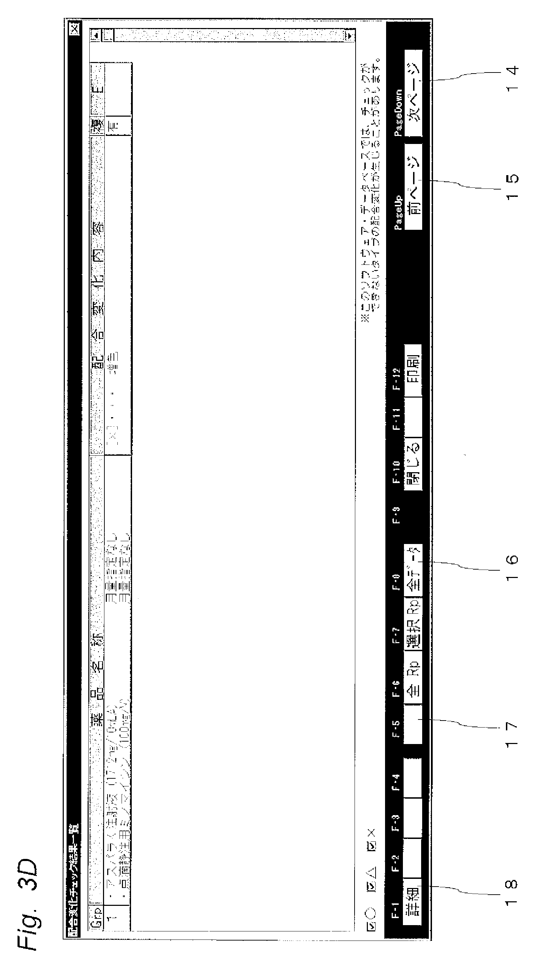

Fig. 3 is a view showing a component information screen;

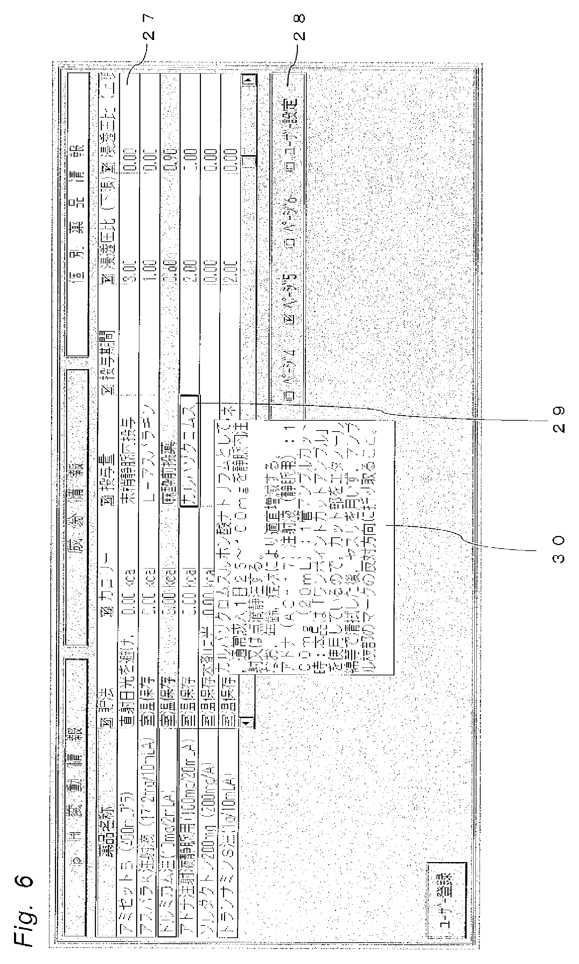

Fig. 6 is a view showing an individual medicine information screen;

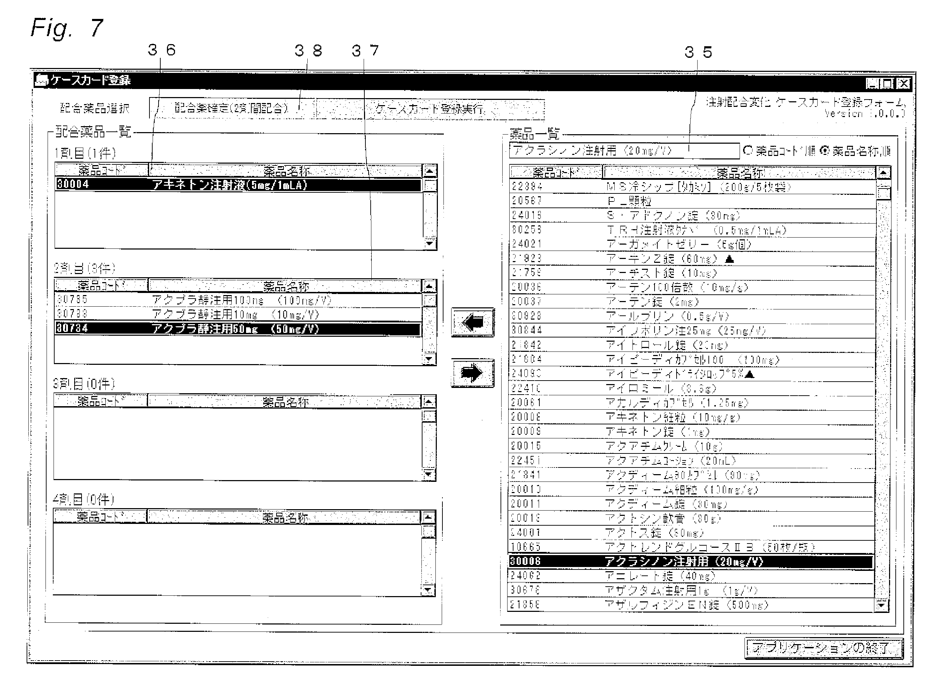

Fig. 7 is a view showing a medicine registration screen;

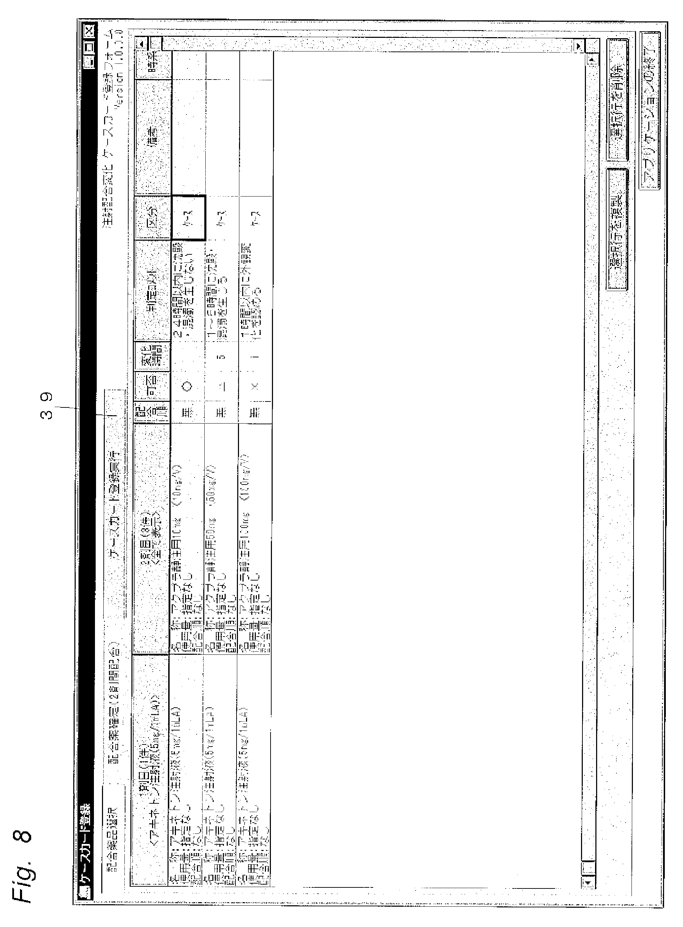

Fig. 8 is a view showing a combined medicine confirmation list screen;

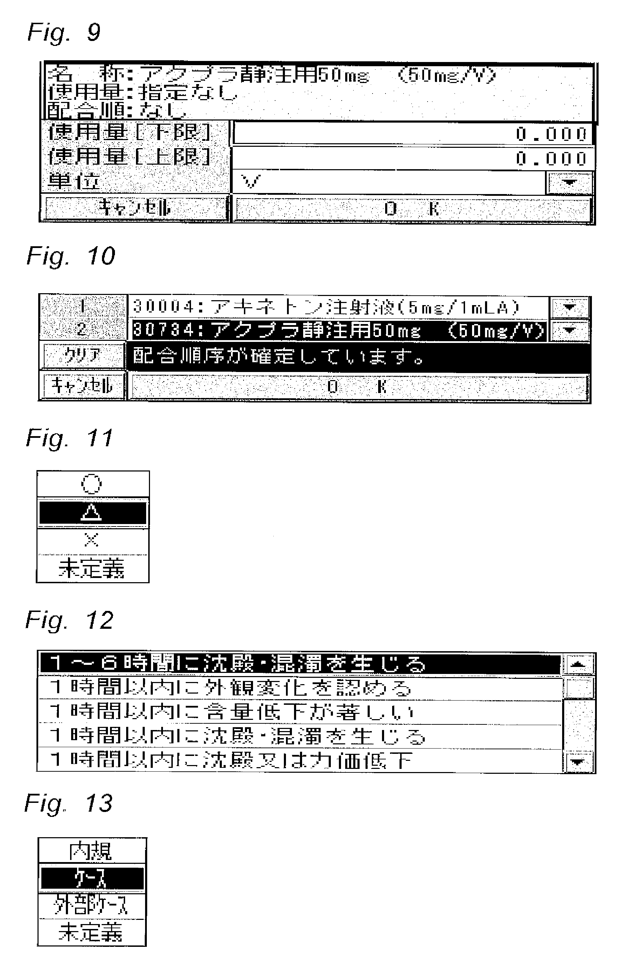

Fig. 9 is a view showing a dosage input screen;
Fig. 10 is a view showing a combination order setting screen;
Fig. 11 is a view showing an adequacy setting screen;
Fig. 12 is a view showing a judgment comment setting screen;
Fig. 13 is a view showing a category setting screen;

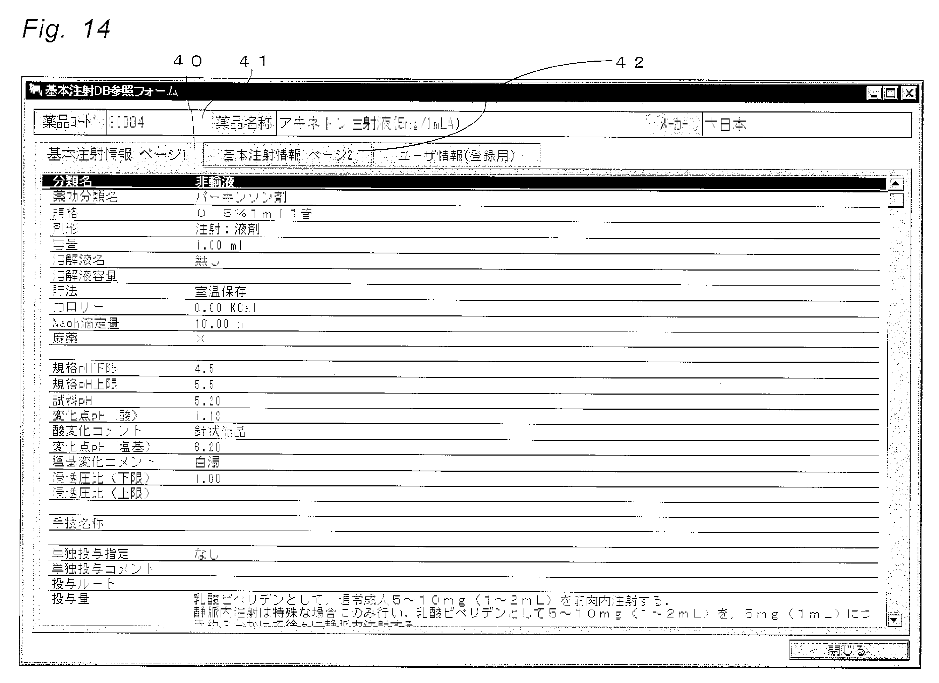

Fig. 14 is a view showing basic injection medicine information screen;

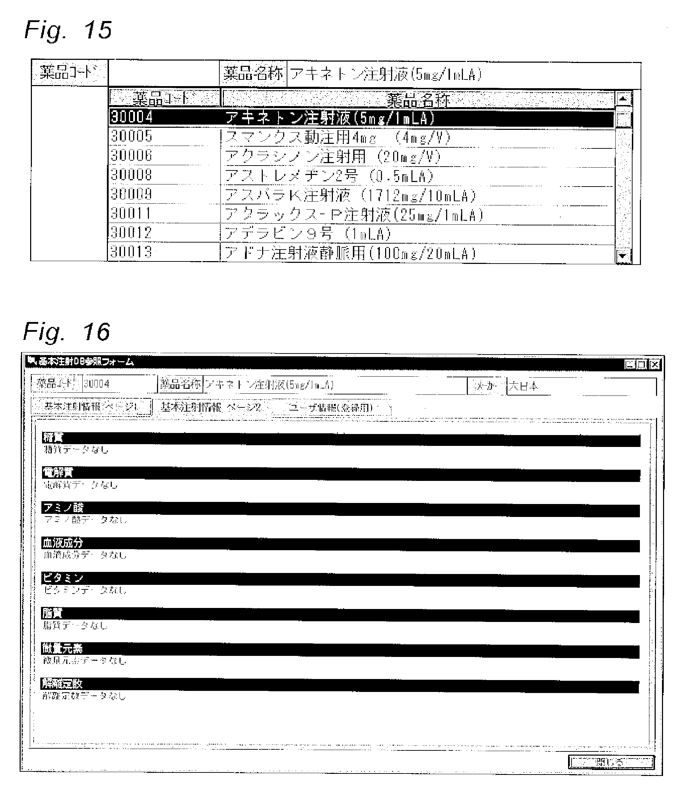

Fig. 15 is a view showing a medicine code selection screen;
Fig. 16 is a view showing a component information screen;

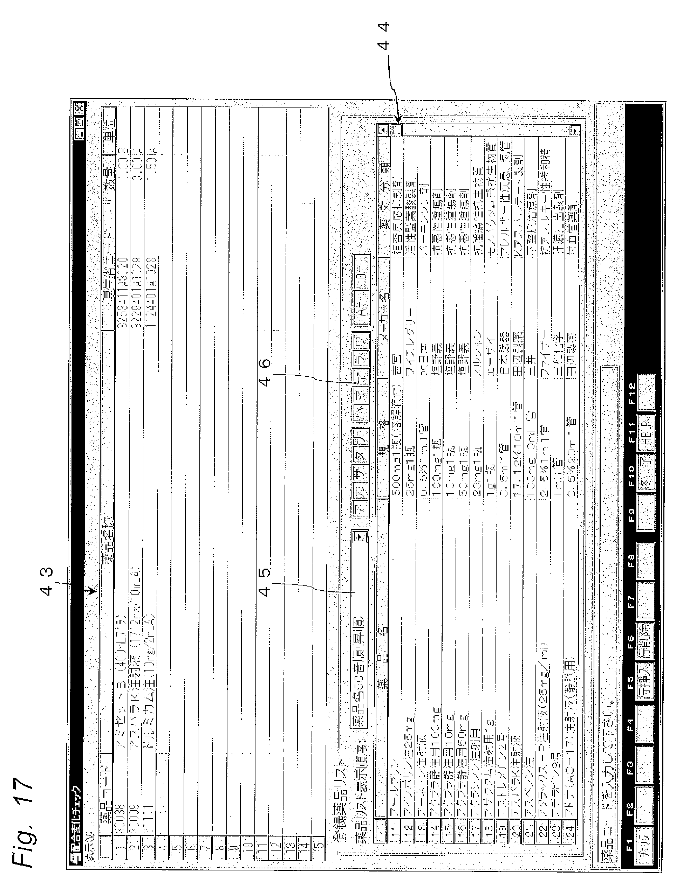

Fig. 17 is a view showing a simple prescription input screen;

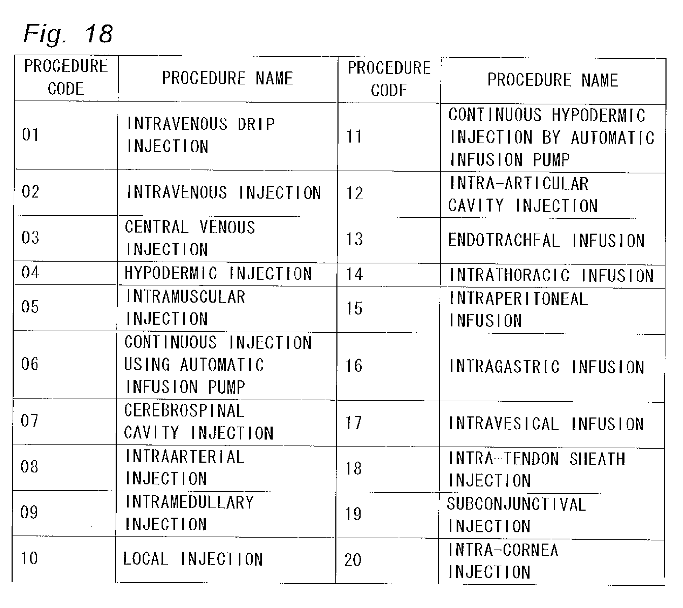

Fig. 18 is a view showing a list of procedure names and code numbers; and

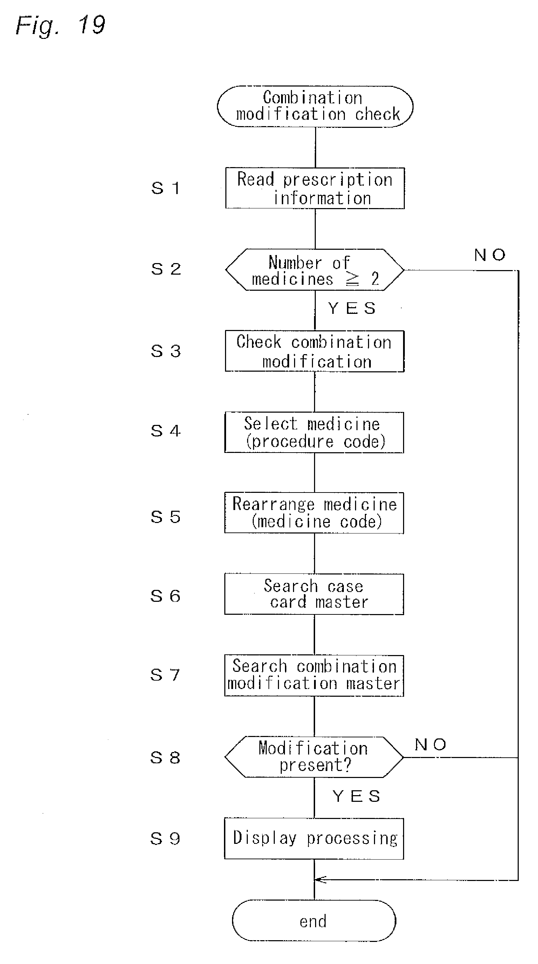

Fig. 19 is a flowchart showing control contents of the medicine management system.

### DETAILED DESCRIPTION OF THE INVENTION

Hereinbelow, the embodiment of the present invention will be described with reference to the accompanying drawings.

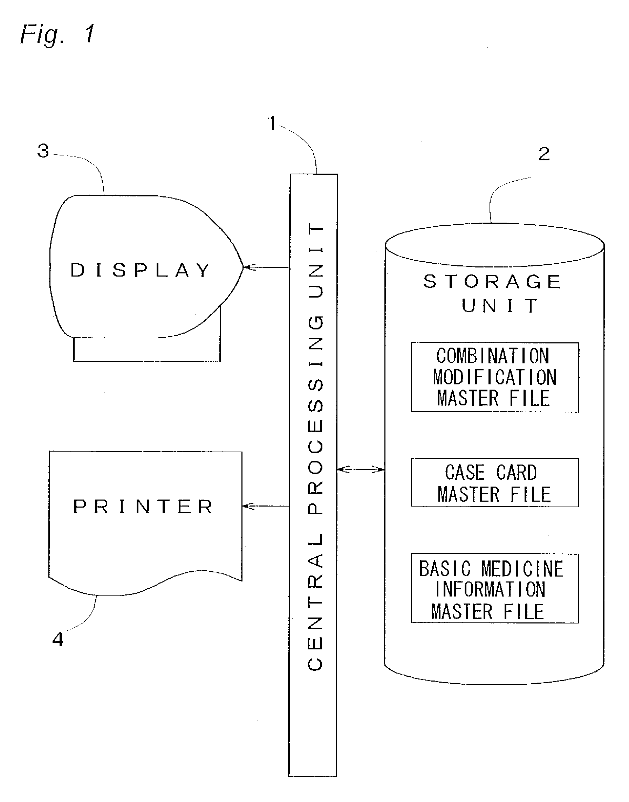

Fig. 1 shows a medicine management system according to the present, embodiment. The system is structured such that in a computer installed in a pharmaceutica department and the like, a central processing unit 1 (CPU) can judge combination adequacy while displaying specified information on a display 3 based on information stored in a storage unit 2. The judgment results of combination adequacy and the like can be printed by a printer 4.

In the storage unit 2, which may be formed from various storage media such as hard discs, a combination modification master, a case card master, a basic medicine information master and the like are stored.

In the combination modification master, combination modification information (researched in documents and the like) indicating what kind of change occurs when two or more medicines are combined is stored in advance (the combination modification information cannot be updated). More specifically, medicines are rearranged according to medicine codes associated with respective medicines, and the combination modification information is stored for every combination of medicines obtained. For example, when medicines A (001), B (003) and C (002) (parenthetical numbers are medicine codes) are combined, combination modification information for a combination in this order (e.g., "no problem", "clouded after combination of A and C", etc.) is stored. The order of rearranged medicine codes has nothing to do with the combination order, and the combination order is not necessarily identical when combined medicines are different. In the case card information stored in the case card master, combination modification information based on clinical examples of combination modification obtained in the medical scenes and the like is stored in the same format as the combination modification master. While the combination modification master cannot be updated, the case card information can be updated freely based on the combination results on the spot. The basic medicine information master includes medicine codes, medicine names, manufacturer names, therapeutic categories and procedure codes. Herein, the procedure codes are codes, as shown in Fig. 18, corresponding to procedure names indicating which region of a body an injection is applied to. When there are a plurality of injections applied to the same region, these injections arc normally combined together before being applied.

In the display 3, a prescription input/audit screen, a combination modification check by medicine result screen, a combination modification information by medicine screen, a basic medicine information screen, a component information screen, a case card medicine selection screen, a case card detailed registration screen and the like are displayed.

The printer 4 prints the combination modification information by medicine and the like displayed on the display 3.

The central processing unit 1 makes reference to the combination modification information stored in the storage unit 2 based on prescription information, and instructs execution of judgment, display, print of combination adequacy and the like as shown below.

The operation of the medicine management system is now described following the flowchart in Fig. 19.

First, prescription information is read from a host computer (step S1). Then, according to the read prescription information, it is judged whether or not two or more medicines are prescribed to each client (step S2). If two or more medicines are prescribed, then combination modification is checked (step S3). As a result, as shown in later-described Fig. 3D, the result or combination check is displayed by a list.

In the combination modification check, medicines injected at the same time, by the same method and to the same region are selected for every patient according to the procedure code allotted to each medicine (step S4). Then, the selected medicines are rearranged according to the medicine codes (step S5). Then, the case card master is searched based only on a combination (order) of medicines stored in the arranged order (step S6), and then the combination modification master is searched (step S7). When similar registration information is found in both the masters, priority is given to the registration information in the case card master. If it is judged that combination modification is present among the combination of the medicines (step S8), then specified data is displayed on the display 3 as described later (step S9).

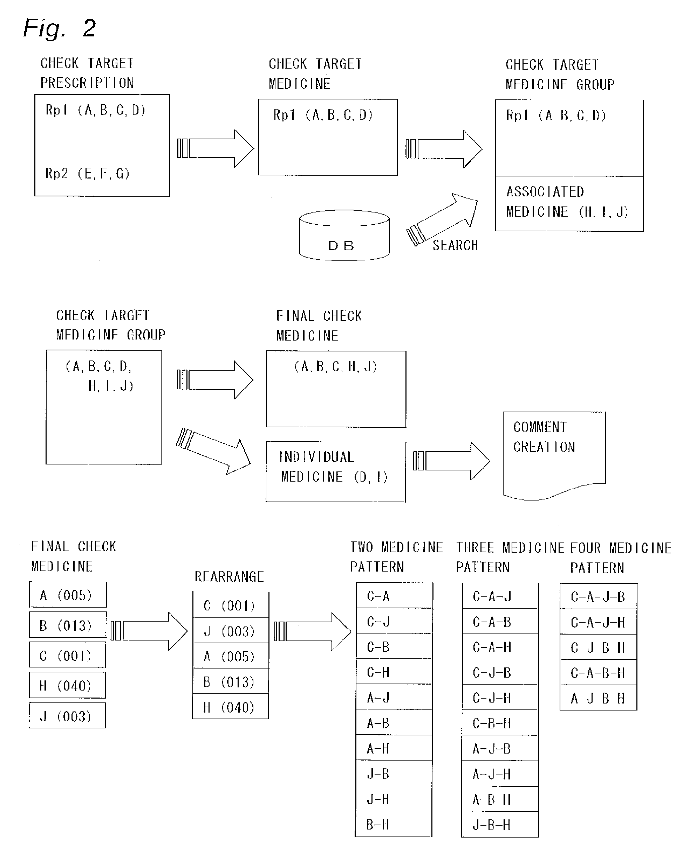

More specifically, as shown in Fig. 2, first, prescription information (Rp1, Rp2...) requiring combination check is sequentially picked up as check target medicines. Then, it is searched whether or not a patient to which the picked-up chock target medicines is prescribed has other medicines (those targeted as injection and drip infusion) prescribed on the same day, and if the patient has other medicines, then these medicines are also included in a check target medicine group. Herein, medicines subject to individual administration are distinguished from the check target medicine group, and only the medicines subject to mixed administration undergo modification check for checking whether or not the combination is found in the combination modification master and the case card master. The mixed administration is determined by "whether or not the medicine is an injection applied to the same region according to the procedure code in the basic medicine information master. This allows further narrow-down of medicines subjected to search.

It is to be noted that when a plurality of prescriptions are issued to a certain patient, the combination modification check should preferably be performed whenever a prescription is issued. For example, in the case where a patient is urgently admitted to hospital due to an accident and the like, the patient's condition varies from hour to hour, and so prescriptions are issued according to the varied conditions. In this case, whenever a prescription is issued, the combination modification check is performed. When medicines are finally administered to the patient, the combination modification check should be performed based on the information of all the prescriptions of the patient. This allows execution of two or three combination modification checks.

According to the combination check method, when, for example, two medicines C and A are mixed, consideration needs to be given only to the combination C-A and not to the combination A--C, that is the combination in different combination order. Therefore, the number of searches in the combination modification master and the case card master can be reduced, thereby allowing the effective combination check. More specifically, when check targets are five medicines, 200 searches are necessary in a conventional method, whereas in the present invention, only 25 searches are needed.

Moreover, according to the combination check method, the combination check of 100 prescriptions can be performed in 30 minutes. The actual number of combinations which cause combination modification is 20 to 30 prescriptions. Without the use of the case card master, the cases which potentially bear the result showing the presence of combination modification can be narrowed down only to 100 to 200 prescriptions. With use of the case card master, the search is based on the information obtained at actual medical scenes so that the information has high reliability and the search combinations can be narrowed down to 50 to 60 prescriptions. This allows final confirmation of the combination check to be performed at short times. It is naturally understood that as information in the case card master is reinforced, the search target combinations are further narrowed down and closer to an actual value.

Moreover, the combination check is performed after usual business hours such as daring night hours, and only those having error (combination modification) are tabulated and listed by batch processing so that spare time can be effectively used. The tabulated result may be printed out. The tome zone in which the combination check is performed can be set freely, e.g., day time, in conformity to the needs of users.

Fig. 3A shows a tabulated result, i.e., an injection audit list screen shown on the display 3. On the infection audit list screen, those having error in the combination check are displayed by a list. A display content 29 denotes a prescription number (prescription No.), an order number, a prescription category, a ward, an administration date, a patient ID, and a patient name. It is to be understood that the content is not limited to these.

When a read button 10 is clicked on the injection audit list screen, the screen is updated and latest data is displayed. Further on the injection audit list scr een, when either one of lines is selected and an audit button 11 is clicked, or the line is double-clicked, all the medicines prescribed to a certain patient are displayed on a prescription input screen shown in Fig. 3B and Fig. 3C. When there is combination modification (shown by an arrow in Fig. 3C) as a result of the combination check, "combination modification error" 12 button is highlighted in red. Herein, when a "combination modification information" button 13 is clicked, the search result of the combination modification master is displayed as a combination modification result list screen shown in Fig. 3D. By clicking a "next page" button 14 (not visible as it is highlighted in reverse video in Fig. 3D), the combination modification result after the displayed medicine is displayed. By clicking a "previous page" button 15 (not visible as it is highlighted in reverse video in Fig. 3D), the combination modification result before the displayed medicine is displayed. By clicking an "all data" button 16, all the combination modification check results of the pertinent prescriptions are displayed. By clicking on "individual data" button 17, the display content is switched to data per medicine.

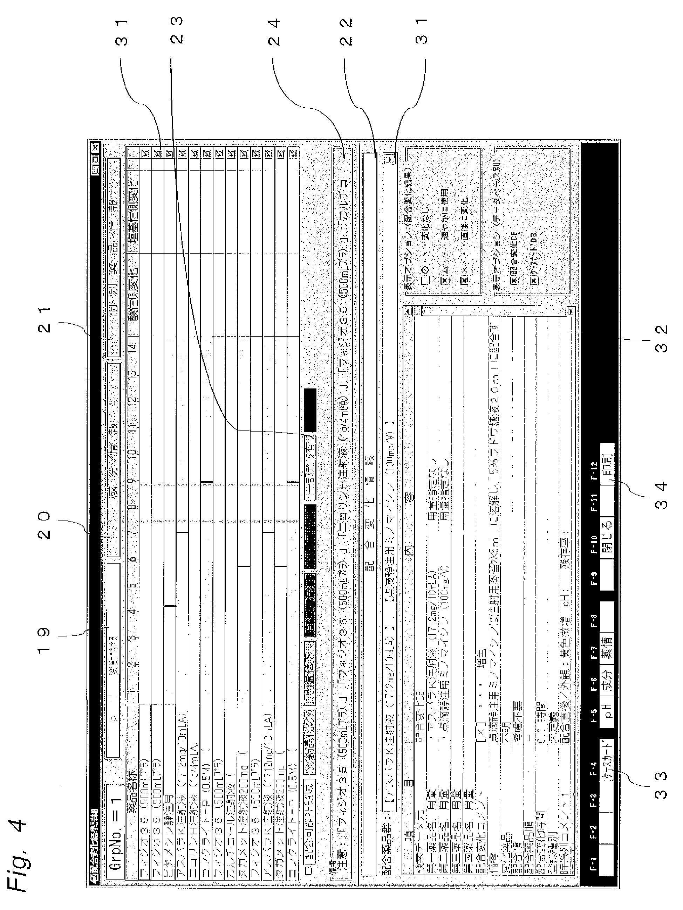

Moreover, by clicking a "detail" button 18, a combination modification Information screen shown in Fig. 4 is displayed. This screen includes a medicine "pH change information" button 19 and a "component information" button 20 or an "individual medicine information" button 21 on the upper side of the screen and a "combination modification information" column 22 on the lower side, each of which are color graphic-displayed.

In the PH change information screen shown in Fig. 4, in each medicine, a region reflecting a change due to the pH change is distinguished by using different colors according to the contents of the change (an unchanged region is displayed in white and display colors can be changed. The difference in color cannot be distinguished in the drawing). When no change is observed after 10ml solution is all titrated on the acid side and the alkaline side, a "partial data available" 23 is displayed in faint blue color. A sample pH is displayed by a black vertical bar. When combination modification occurs, the contents of the change are displayed on the right-hand section in a graph, and by right-clicking this section, all the overspilled characters become recognizable. Moreover, among all the medicines, the highest value of acid-side change points is identified by a red line while the lowest value of alkaline-side change points is identified by a blue line. These regions are the regions of pH values which do not cause any change in all the medicines. It is to be noted that by deselecting a right-side check button on the screen, (all the check buttons are ON as default), the graphs are changed to a faint color and can be made out of the target of red and blue fines. For confirming an pH value of a graphical representation section, right-clicking a section desired to be confirmed allows the pH value to be displayed on the upper section of a mouse. When information on the change point is not included in selected medicine information, a comment is shown in an "remark" column 24 on the lower side of the screen as a caution.

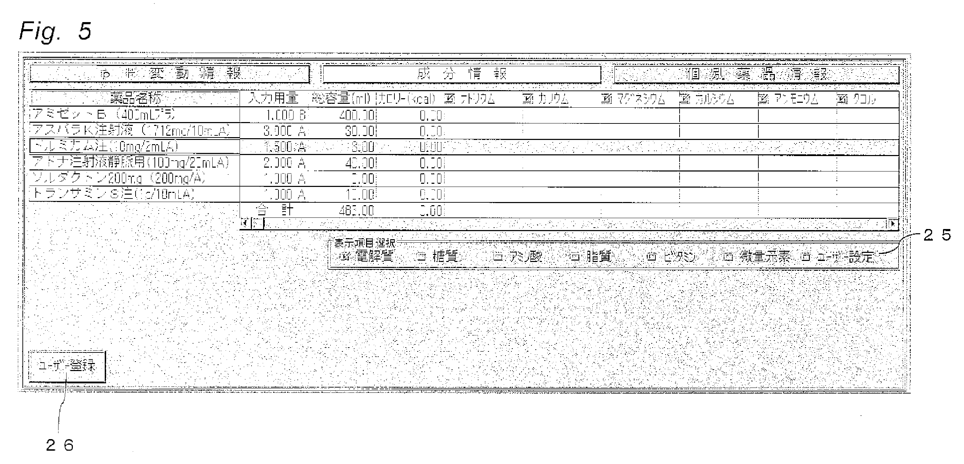

Fig. 5 shows a component information screen. The screen shows a list of component values of displayed medicines such as inputted quantities, total quantities and calories as information quoted from the medicine master. Items other than "inputted dosage" , "total quantity" and "calorie" can be displayed if selected. When each item (category name) is checked in a "select display item" column 25 in the lower side of the table, items belonging to the category are displayed. If there are nine or more display items, horizontal scroll allows these items to be displayed. By checking an item and clicking a "user registration" button 26 on the left-side of the screen, the selected item is registered as a "user setting" item, and when the "user setting" in a display item selection section is checked, the registered item is displayed. Among items, "sodium" and "potassium" in particular have all acceptable daily dosage limits, and so an accident can be prevented from occurring by calculating the daily sum of all the medicines administered to a certain patient and comparing the sum with the dosage limits of these items.

Fig. 6 shows all individual medicine information screen. The screen shows a list of each item in the basic medicine information of each medicines which is selected and read from the medicine master. It is possible to select and display the desired items or display the user's registered items in an item check button column 2 / on the upper side of the screen and in a page select column 28 on the lower side of the screen as well as the display of the content information. Also, by clicking the display content 29, it is possible to pop up detailed information 30 registered in the medicine master.

Moreover, as shown in Fig. 4, the combination modification information screen is displayed on the lower half section of the combination modification detailed screen. By clicking a combination medicine group column 31, a list of all the combinations of the medicines displayed in the medicine name column 32 in the pH change information can be displayed. When an arbitrary medicine group is selected from the displayed list of medicine combinations, the registered combination modification information is displayed on the lower side. The combination modification information on the screen can be rewritten and registered in the case card. More particularly, while the combination modification information stored in the combination modification master is legitimate information disclosed in documents and the like, the information is often changed to a degree in practical operation, and so the updated information is registered in the case card master and given precedence in use. By inputting combination modification comments, time-series comments and the like, and clicking a "case card" button 33, the updated information is automatically inputted into the case card. For printing combination modification information including "pH change information (graphical representation)", "mixed medicine information (total infusion and total calories), "combination modification information", "individual medicine information (selectable from individual administration comments, precautions for application and the like), a "print" button 34 on the lower right-side of the screen should be clicked, it is to be noted that using a display option ("combination modification result" and "combined medicine number") on the lower right-side of the screen allows the number of groups displayed in the combination modification medicine group to be restricted. Moreover, as for a medicine group selected in the lower section of the screen, the color of the upper medicine list section can be changed (to faint green color for example) to clarify a medicine displayed in the combination modification information section.

Moreover, for registering combination modification information on a new combination of medicines not yet registered in the combination modification master in the case card, a medicine registration screen shown in Fig. 7 should be opened and medicines should be selected from a displayed medicine list 35 in the order of the first medicine 35, the second medicine 36 and so on.

Fig. / shows the state that the medicines in the medicine list 35 are sorted in the order of medicine names (sorting in the order of medicine codes is also possible), and one medicine is selected for a first medicine column 36 while three medicines are selected for a second medicine column 37. Herein, by clicking a "combined medicine confirmation" tab 38 on the upper side of the screen, all the combinations of the selected medicines, (in this case, 1 × 3= 3 combinations) are displayed by a list as a combined medicine confirmation list screen as shown in Fig. 8. When there is registered combination modification information, the contents of the information are displayed, whereas when there is no registered combination modification information, necessary information including "dosage", "combination order", "adequacy", "change time" and "judgment comment" should be inputted and then a "case card registration execute" tab 39 should be clicked to perform automatic registration.

For inputting the details of combination information, double-clicking or right-clicking the pertinent section makes it possible to input necessary information corresponding to each item. For example, in the "dosage" section, a dosage input screen shown in Fig. 9 is opened, so that a lower limit, an upper limit and a unit of the dosage can be inputted. In the "combination order" section, a combination order setting screen shown in Fig. 10 is opened, so that a combination order can be determined by clicking medicine columns in sequence. In the "adequacy" (judging adequacy of combination) section, a adequacy setting screen shown in Fig. 11 is opened, so that "○" (adequate), "Δ" (conditionally adequate) and "×" (inadequate) can be selected. In the section of "judgment comment", a judgment comment setting screen shown in Fig. 12 is opened, so that pertinent comment can be selected. In the "category" section, a category setting screen shown in Fig. 13 is opened, so that a pertinent category can be scdected. It is to be noted that other items such as "remark" and "time-series comment" can be accessed by selecting the pertinent items on the combined medicine confirmation list screen and inputting data through a keyboard and the like. In the case of inputting combination modification information on a combination of identical medicine codes with different dosages, a "copy selected line" bottom can be used to save the trouble so that only dosage should be changed. Moreover, when there is an unnecessary combination of medicine codes, the combination can be deleted by selecting the pertinent line and clicking a "delete selected line" bottom. It is to be noted that when an identical combination of medicines is present in the combined medicine confirmation list, a massage window informing that the combination cannot be registered is popped up.

It is to be noted that the information on each medicine can be checked by displaying a basic injection medicine information screen shown in Fig. 14 (in this case, the screen in the state that a "basic information 1" tab 40 is clicked is displayed), and inputting each medicine code. When a medicine code is unknown, a medicine code selection screen shown in Fig. 15 can be displayed by double-clicking a medicine code column 41 and a desired medicine can be selected. On the basic injection medicine information screen, clicking a "basic information 2" tab 42 displays a component information screen shown in Fig. 16, which allows confirmation of component information. The information displayed on each screen is read from the medicine master.

Although in this embodiment, the prescription data for checking combination modification is automatically read from the host computer, the prescription data can be inputted manually by using a simple prescription input screen shown in Fig. 17.

On the simple prescription input screen, "medicine code", "medicine name", "Ministry of Health and Welfare code", "quantity" and "unit" are displayed (inputs can be made to an underlined item) in a prescription input section 43. In a registered medicine list section 44 on the lower side of the screen, "medicine name", "standard", "manufacturer name" and "therapeutic category" of all the registered medicines are displayed. On the upper side of the list, an "order select" column 45 and an "access" button 46 are displayed. Input operation using the simple prescription input screen allows direct inputs of the "medicine code" from a keyboard (medicine code direction input mode), selection from a "medicine code list" (medicine code list select mode), selection from a "registered medicine list" (registered medicine fist select mode), and selection from a "barcode list" (barcode list input mode). In the medicine code direction input mode, direct inputs of medicine codes by a keyboard makes it possible to read pertinent data (medicine names and Ministry of Health and Welfare codes) from the medicine master, and the data can be displayed. In the medicine code list input mode, the medicine code list can be displayed when a medicine code has not yet been registered in the medicine master or when users do not input a medicine code. In the registered medicine list select mode, the registered medicine list can be displayed and a medicine can be identified from the standard, the manufacturer, the therapeutic category and the like. The medicines in the list are arranged in the "order of the medicine names according to Japanese syllabary", in the "order of manufacturers according to Japanese syllabary", or in the order of therapeutic categories, and the medicines can be rearranged by selecting any one of the orders. The quantity and unit can be inputted into the pertinent columns by a keyboard and the like. In the unit column, a first unit is displayed as a default. If the pertinent medicine has a second unit and a third unit, then every time "space" key is pressed, the unit can be switched in the order of "first unit", "second unit", "third unit", and "first unit". It is to be noted that information inputted by a unit other than the first unit is all converted to information by the first unit, and then the information is subjected to the combination modification check and reflected on a result display screen.

Further, although in this embodiment, database search is performed based on the combinations of medicines rearranged in the order of medicine codes in the storage unit 2, the search may be performed according to a Hush function as shown below.

More particularly, from the medicine name and the medicine code of medicines to be combined, a hush value is obtained for every combination of medicines as combination modification information, and is registered in the combination modification master as a search key. Then, a value matched with the hush value obtained from a combination of medicines to be combined can be searched from the combination modification master. In this case, a hush function should, be the function which returns different values when medicine combinations are different and which returns identical hush values when the combinations are different in order.

More specifically, when a medicine A (medicine code: 123) and a medicine B (medicine code: 456) are combined, adjacent values and a top value and a final value of each medicine are multiplied (medicine A: 1 × 2=02, 2 × 3=06, 1 × 3-03, medicine B: 4 x 5=20, 5 × 6- 30, 4 x 6 = 24). Then, the obtained calculation results are inserted after each numerical values of the respective medicine codes (medicine A; 102206303, Medicine B: 420530624), and the obtained results of the respective medicines are added to obtain a Hush value (102206303+420530624-522736927). According to the thus-obtained Hush value, different values are obtained from combinations of different medicines and identical values are obtained from identical combinations with different combination orders.

Thus, when the Hush function is used, it is not necessary to rearrange the registration order of medicines in the combination modification master or to rearrange medicines to be checked according to the code order. In addition, if registration of medicines in the combination modification master is conducted in the order of combination, it is also possible to omit additional operation to store a combination order field, i.e., a combination order in which combination modification occurs.

Although in this embodiment, particular consideration is not given to the combination order in the case of combining three or more medicines, a "combination order presence flag" field and a "combination order" field may be stored additionally. In the "combination order presence flag" field, data indicating that difference in combination order generates combination modification is stored, while in the "combination order" field, the combination order in the presence of the combination order is stored. Then, when the pertinent record is searched in the "combination modification master" during combination modification check operation, the "combination order presence flag" field is referred to confirm the presence of the combination order, and if the combination order is involved with combination modification, a then the "combination order" field is referred and medicines are rearranged in the combination order, and a comment stating that the combination order is involved with combination modification is displayed. As a result, it becomes possible to automatically identify the combination order, which allows combination error to be prevented from occurring, and allows considerable increase in working efficiency.

Although in this embodiment, numerical numbers are used as medical codes and procedure codes, symbols as well as medicine names and procedure names themselves can also be used as long as the order of medicines can be rearranged according to these codes.

## Claims

1. A medicine management system, comprising:
storage means for storing medicine information on medicines and combination modification information showing change when a plurality of medicines arc combined; and
combination adequacy judging means for judging combination adequacy based on the combination modification information stored in the storage means when two or more medicines are included in information of one or more prescriptions for a certain patient, wherein
the storage means stores medicine codes associated with respective medicines and combination modification information corresponding to a combination of medicine information rearranged according to the medicine codes, and
the combination adequacy judging means rearranges the medicine information according to the medicine codes stored in the storage means when two or more medicines are included in the prescription information, and judges combination adequacy with reference to the combination modification information called from the storage means according to obtained order and combination of the medicine information.

2. A medicine management system, comprising:
storage means for storing medicine information on medicines and combination modification information showing change when a plurality of medicines are combined; and
combination adequacy judging means for judging combination adequacy based on the combination modification information stored in the storage means when two or more medicines are included in prescription information, wherein
the storage moans stores medicine related information on respective medicines, calculates combinations of two or more medicines by a hash function based on the medicine related information, and stores combination modification information for every obtained hash values, and
when the prescription information includes two or more medicines, the combination adequacy judging means calculates hash values based on the medicine related information stored in the storage means and calls corresponding combination modification information from the storage means based on the obtained hash values to judge combination adequacy.

3. The medicine management system as defined in Claim 1 or 2, wherein the storage means stores a master file in which unrewritable combination modification information is stored and a case card file in which combination modification information can be newly stored, and
during judgment of combination adequacy, the combination adequacy judging means judges combination adequacy preferentially based on the combination modification information stored in the case card file.

4. The medicine management system as defined in any one of Claims 1 to 3, further comprising display means for displaying the combination adequacy judged in the combination adequacy judging means, wherein
combination modification information on combinations of all medicines displayed in the display means can be displayed.

5. The medicine management system a s defined in Claim 4, wherein the combination modification information on combinations of all medicines displayed in the display means can be changed and can be newly stored in the case card file.

6. The medicine management system as defined in Claim 4 or 5, wherein after a medicine registration screen allowing a plurality of combined medicines to be registered with respect to each combination unit is displayed on the display means, all combinations among all combination units can be displayed by a list on a combined medicine confirmation screen, and new combination modification information can be inputted and stored in the case card file.

7. The medicine management system as defined in any one of Claims 4 to 6, wherein when a stored combination of medicine information has combination modification due to difference in combination order, the storage means stores the change, and when the pertinent combination is referred, medicine information is rearranged in an appropriate combination order and displayed on the display means.

8. The medicine management system as defined in Claim 1 or 2, wherein when the prescription, information includes two or more medicines, the combination adequacy judging means selects medicines to be simultaneously administered based on procedure codes stored in the storage means before rearranging the medicine information according to the medicine codes.
